Europäisches Patentamt

European Patent Office

Office européen des brevets

(11) Publication number: **0 143 864**
**B1**

## EUROPEAN PATENT SPECIFICATION

(45) Date of publication of patent specification: **22.04.87**

(51) Int. Cl.⁴: **C 07 C 19/08,** C 07 C 17/06

(21) Application number: **83307328.1**

(22) Date of filing: **01.12.83**

(54) Process for the production of 1,1,2-trichloro-2,2-difluoroethane.

| | |
|---|---|
| (43) Date of publication of application:<br>**12.06.85 Bulletin 85/24** | (73) Proprietor: **THE DOW CHEMICAL COMPANY**<br>**2030 Dow Center Abbott Road P.O. Box 1967**<br>**Midland, MI 48640 (US)** |
| (45) Publication of the grant of the patent:<br>**22.04.87 Bulletin 87/17** | (72) Inventor: **Larsen, Eric Russell**<br>**314 W. Meadowbrook**<br>**Midland, MI 48640 (US)**<br>Inventor: **Ecker, Ernest Levern**<br>**346 Dopp Road**<br>**Midland, MI 48640 (US)** |
| (84) Designated Contracting States:<br>**CH DE FR GB LI NL** | |
| (56) References cited:<br><br>**None** | (74) Representative: **Smart, Peter John et al**<br>**W.H. BECK, GREENER & CO 7 Stone Buildings**<br>**Lincoln's Inn**<br>**London WC2A 3SZ (GB)** |

Courier Press, Leamington Spa, England.

## Description

This invention relates to a new method for the preparation of 1,1,2-trichloro-2,2-difluoroethane.

The compound 1,1,2-trichloro-2,2-difluoroethane has been prepared in the past by a variety of methods. These methods include the thermal chlorination of $CClF_2CH_3$ and $CHF_2CH_3$, the reduction of $CClF_2CCl_3$ with isopropanol (2-propanol), and the fluorination of $CCl_3CHCl_2$ with antimony fluoride. Except for the fluorination of $CCl_3CHCl_2$ and the reduction of $CClF_2CCl_3$, 1,1,2-trichloro-2,2-difluoroethane was not the major product and yields were generally poor. The fluorination of $CCl_3CHCl_2$ suffers as a method of preparing 1,1,2-trichloro-2,2-difluoroethane because it involves corrosive HF and produces a hazardous, antimony-containing waste stream. The reduction of $CClF_2CCl_3$ is not a satisfactory method of making 1,1,2-trichloro-2,2-difluoroethane, due to the fact that $CClF_2CCl_3$ is simply 1,1,2-trichloro-2,2-difluoroethane which has been overchlorinated.

In view of the deficiencies of the aforementioned known processes, it would be highly desirable to provide a process having a high selectivity to 1,1,2-trichloro-2,2-difluoroethane without having the problems associated with the methods of the prior art.

The present invention is such a highly selective process for producing 1,1,2-trichloro-2,2-difluoroethane. The process involves contacting 1,1-difluoroethylene with a chlorinating agent in the presence of a free radical initiator under conditions such that $CF_2ClCHCl_2$ is selectively obtained. Said selectivity is preferably greater than 63 mole percent based on 1,1-difluoroethylene. These selectivities are surprising in view of the results obtained by the techniques of the prior art.

The compound 1,1,2-trichloro-2,2-difluoroethane is a valuable intermediate for the preparation of highly desired chemicals. For example, 1,1,2-trichloro-2,2-difluoroethane may be combined with KOH and an alcohol to produce a halogenated ether via the method disclosed in British Patent Specification 523,449. Further, 1,1,2-trichloro-2,2-difluoroethane may be dehydrochlorinated to 1,1-dichloro-2,2-difluoroethylene, which in turn may be contacted with methanol in the presence of alkali to produce 2,2-dichloro-1,1-difluoroethyl methyl ether, as shown in U.S. Patent 3,264,356.

In the practice of the present invention it is essential to employ 1,1-difluoroethylene, a chlorinating agent, and a free radical initiator. The compound 1,1-difluoroethylene is well-known, is commercially available, and can be prepared by a number of known methods.

A suitable halogen or any agent that is capable of generating chlorine atoms, such as a suitable chlorine-containing solid or liquid, can be used as the chlorinating agent. The preferred chlorinating agent is chlorine. Advantageously, the chlorinating agent is supplied in an amount such that an optimum amount of 1,1,2-trichloro-2,2-difluoroethane is produced. Typically, the molar feed ratio of $Cl_2$ to 1,1-difluoroethylene is from 1.2 to 2.5, preferably this ratio is from 1.7 to 2.1. A lower or higher ratio may be employed, but low $Cl_2$/1,1-difluoroethylene molar feed ratios lead to excessive accumulation of $CF_2ClCH_2Cl$, while high ratios lead to accumulation of $CF_2ClCCl_3$ and a reduction in the selectivity of the process.

Suitable free radical initiators include catalysts, such as peroxides, and electromagnetic radiation. Electromagnetic radiation is the preferred free radical initiator. Any form of electromagnetic radiation which effects the instant chemical reaction may be employed, such as, for example, radiation in the visible and ultraviolet regions. Ultraviolet light is the most preferred free radical initiator. The output of the reaction system is a function of the light intensity, i.e., the rate of free radical formation, and the rate of addition of the reactants.

The chlorination of 1,1-difluoroethylene is advantageously conducted in a liquid reaction medium at a temperature which is between −20°C and 100°C. Preferably, the chlorination will be conducted between 0°C and 40°C. Ordinarily, the reaction will proceed readily at atmospheric or higher pressure; subatmospheric pressures can be employed if desired. Preferably the chlorination is conducted at atmospheric pressure.

The reaction preferably takes place in the presence of a liquid reaction medium. The liquid reaction medium may be any suitable liquid which does not detract from the efficacy of the reaction. The liquid reaction medium preferably contains a molar excess of $CH_2ClCH_2Cl$ with respect to $CF_2ClCHCl_2$, and the molar ratio of the former to the latter is typically 1.5 or greater. Preferably, the molar ratio of $CF_2ClCH_2Cl$ to $CF_2ClCHCl_2$ in the liquid reaction medium will be from 2 to 10. Most preferably, this ratio will be from 3.5 to 10. A ratio of 1.5 will result in a selectivity to 1,1,2-trichloro-2,2-difluoroethane of approximately 63 percent. Ratios which are greater than 10 result in excessive recycle of $CF_2ClCH_2Cl$ in exchange for a small small gain in selectivity. Preferably, a selectivity of 70 percent or more is obtained; most preferably a selectivity of 80 percent or greater is obtained. The term selectivity, when applied to the process of the present invention, means selectivity of 1,1,2-trichloro-2,2-difluoroethane, based on 1,1-difluoroethylene fed to the reaction vessel, and is calculated using the following equation:

$$\text{Selectivity} = (100)(Y)/(Y+Z);$$

wherein Y is moles of $CF_2ClCHCl_2$ in the product stream, and wherein Z is moles of $CF_2ClCCl_3$ in the product stream.

In a typical embodiment of the invention, the reaction vessel is charged with either $CF_2ClCH_2Cl$ or a mixture, from a previous run, of $CF_2ClCH_2Cl$, $CF_2ClCHCl_2$, and $CF_2ClCCl_3$. The reaction vessel contents are irradiated by free-radical-initiating electromagnetic radiation, and then gaseous chlorine and 1,1-difluoroethylene are fed simultaneously in separate feed streams having controlled flow rates to the reaction vessel through gas dispersion tubes. As the reaction proceeds, the liquid level rises in the reactor, gaseous HCl evolves from the liquid reaction medium, and the reactor is cooled in order to minimize vaporization of the relatively low boiling contents of the reactor ($CF_2ClCH_2Cl$ boils at about 47°C), said vaporization being promoted by the heat of the exothermic reaction and the thermal energy given off by typical lamps used to generate free-radical-initiating electromagnetic radiation.

Prior to the point in time at which the reactor will overflow into its overflow line, which is connected to a reboiler of a distillation column, said reboiler is charged with $CF_2ClCH_2Cl$ and heated to achieve a steady reflux. When the reactor overflows to the reboiler, the distillation column proceeds to separate the mixture in the reboiler. The temperature at the top of the distillation column is chosen so that the distillate is essentially poure $CF_2ClCH_2Cl$. A condenser at the top of the column condenses the vapors of the halogenated organic compounds. The condensate from the condenser is split into two streams; one for reflux to the distillation column and one as a recycle stream to the reactor.

The liquid in the reboiler becomes richer in 1,1,2-trichloro-2,2-difluoroethane and $CF_2ClCCl_3$ as the distillation progresses. This liquid may be withdrawn for purification, with a return of $CF_2ClCH_2Cl$ to the reboiler. Alternatively, when the amount of 1,1,2-trichloro-2,2-difluoroethane in the reboiler becomes sufficiently high, the chlorination may be halted and the distillation system can be used to purify the mixture. When, after the purification is complete, the fraction rich in $CF_2ClCH_2Cl$ can be returned to the reboiler, the chlorination may be restarted, and the cycle can be repeated as desired.

The following example is given to illustrate the invention and should not be construed as limiting its scope. All percentages in the example are by weight unless otherwise indicated.

## Example 1

The process equipment includes:

(a) a 2.5 liter glass reaction vessel equipped with gas dispersion tubes at its lower end, a bottom drain, and a cooling means;

(b) a one-liter, 3-necked glass vessel (reboiler) equipped with a bottom drain, a thermocouple, and a heating means;

(c) a 1" × 18" glass distillation column packed with $\frac{1}{4}$" glass helices, and equipped with a thermocouple at its top and a vacuum jacket;

(d) a condensing means with an associated fraction-splitting means; and

(e) a 450 watt Hanovia® medium pressure, quartz, mercury vapor lamp.

The process equipment is assembled so that the packed distillation column sits above the one-liter vessel. The condensing means with associated fraction-splitting means sits above the distillation column.

The reaction vessel is placed so that any liquid overflow from it will flow by gravity to the one-liter vessel. The source of electromagnetic radiation is placed so that it will irradiate the contents of the reaction vessel.

The process is initiated by charging the one-liter vessel with 3.83 moles of $CF_2ClCH_2Cl$ and 0.13 moles of $CF_2ClCHCl_2$. The reaction vessel is charged with 20.5 moles of $CF_2ClCH_2Cl$, 3.41 moles of $CF_2ClCHCl_2$, and 0.35 moles of $CF_2ClCCl_3$. The one-liter vessel is heated so that a steady reflux is achieved through the distillation column. The lamp is placed approximately six inches (152 mm) away from the reaction vessel and is turned on.

At this point the gaseous reactants, chlorine and 1,1-difluoroethylene, are fed to the lower portion of the reaction vessel through the gas dispersion tubes. The molar feed ratio of $Cl_2$ to 1,1-difluoroethylene is 1.90. During a period of 29.35 hours 43.5 moles of $Cl_2$ and 22.9 moles of 1,1-difluoroethylene pass into the reaction vessel. During this same period, distillate from the one-liter vessel is passed into the reaction vessel at approximately 2.6 ml/min. The contents of the reaction vessel are clear, as opposed to being an undesirable yellow-green color which would indicate an excessive rate of chlorine addition.

The volume of liquid in the reaction vessel increases as the reaction progresses. The liquid overflows into the one-liter vessel through a line which contains a vapor seal. Gaseous HCl by-product is taken off the top of the reaction vessel and is routed to the condensing means. The energy being supplied to the one-liter vessel via the heating means causes the overflow liquid to be distilled. A portion of the distillate is returned from the condensing means to the distillation column and the remaining portion is forwarded to the reaction vessel. The temperature at the top of the distillation column is controlled at 45—46°C in order to provide a distillate which is greater than 95 mole percent $CF_2ClCH_2Cl$. This allows the liquid in the one-liter vessel to become enriched in 1,1,2-trichloro-2,2-difluoroethane and $CF_2ClCCl_3$.

The one-liter vessel is partially emptied whenever it becomes nearly full and the removed mixture is partially separated into its components. The fraction rich in $CF_2ClCH_2Cl$ is returned to the one-liter vessel. For the total period of the run, 5.30 moles of $CF_2ClCH_2Cl$ and 0.25 moles of $CF_2CHCl_2$ are returned to the one-liter vessel.

The total weight of all materials added to the system is 9.260 kg, of which 9.147 kg remain at the end of the run. The molar amounts, according to gas-liquid chromatographic analysis, of materials added to the system and recovered therefrom for the run are shown in Table I.

3

TABLE I
Mass Balance

| Component | Moles In | Moles Out |
|---|---|---|
| $CF_2=CH_2$ | 22.9 | — |
| $Cl_2$ | 43.49 | — |
| $CF_2ClCCl_3$ | 0.35 | 2.35 |
| $CF_2ClCHCl_2$ | 3.8 | 17.6 |
| $CF_2ClCH_2Cl$ | 29.6 | 35.5 |
| HCl | — | 20.5 |
| Total Organic | 56.65 | 55.45 |

Organic recovery = 98 mole percent.
Calculated Yield of 1,1,2-trichloro-2,2-difluoroethane = 82.8 mole percent based on $CF_2=CH_2$ fed.
Selectivity = 87.3 mole percent.
Conversion of $CF_2=CH_2$ — 100 percent.

**Claims**

1. A process for the production of 1,1,2-trichloro-2,2-difluoroethane comprising contacting 1,1-difluoroethylene with a chlorinating agent in the presence of a free radical initiator under conditions such that 1,1,2-trichloro-2,2-difluoroethane is obtained.

2. A process as claimed in Claim 1, wherein the chlorinating agent is chlorine.

3. A process as claimed in Claim 1 or Claim 2 wherein the free radical initiator is electromagnetic radiation.

4. A process as claimed in any preceding claim wherein the chlorinating agent and 1,1-difluoroethylene are contacted in a liquid reaction medium.

5. A process as claimed in any preceding claim wherein the temperature of the liquid reaction medium is between −20°C and 100°C.

6. A process as claimed in Claim 4, wherein the liquid reaction medium comprises $CF_2ClCH_2Cl$ and $CF_2ClCHCl_2$ in a molar ratio which is at least 1.5 of the former to 1 of the latter, and wherein the molar feed ratio of the chlorinating agent expressed as available chlorine to 1,1-difluoroethylene is from 1.2 to 2.5.

7. A process as claimed in any one of Claims 4 to 6 wherein the molar feed ratio of the chlorinating agent expressed as available chlorine to 1,1-difluoroethylene is from 1.7 to 2.1, the temperature of the liquid reaction medium is from 0°C to 40°C, the liquid reaction medium comprises $CF_2ClCH_2Cl$ and $CF_2ClCHCl_2$ in a molar ratio of from 2 to 10, and the free radical initiator is ultraviolet light.

8. A process for the production of 1,1,2-trichloro-2,2-difluoroethane comprising contacting 1,1-difluoroethylene with chlorine in the presence of ultraviolet light at a temperature of from about −20°C to about 100°C in a liquid reaction medium comprising $CF_2ClCH_2Cl$ and $CF_2ClCHCl_2$ wherein the molar ratio of $CF_2ClCH_2Cl$ to $CF_2ClCHCl_2$ is at least about 1.5:1, thereby selectively producing 1,1,2-trichloro-2,2-difluoroethane.

9. A process as claimed in any one of Claims 1 to 8 wherein a portion of the liquid reaction medium is removed continuously or intermittently during the course of the reaction and a fraction rich in 1,1,2-trichloro-2,2-difluoroethane and a fraction rich in $CF_2ClCH_2Cl$ are obtained therefrom and wherein the fraction rich in $CF_2ClCH_2Cl$ is recycled to the liquid reaction medium.

10. A process as claimed in any preceding claim wherein the 1,1,2-trichloro-2,2-difluoroethane is obtained with a selectivity of greater than 63 mole percent.

11. A process as claimed in Claim 10 wherein the selectivity is at least 80 mole percent.

**Patentansprüche**

1. Verfahren zum Herstellen von 1,1,2-Trichlor-2,2-difluorethan, gekennzeichnet durch Chlorieren von 1,1-Difluorethylen mit einem Chlorierungsmittel in Gegenwart eines freie Radikale erzeugenden Starters unter Bedingungen, bei denen 1,1,2-Trichlor-2,2-difluorethan erhalten wird.

2. Verfahren nach Anspruch 1, dadurch gekennzeichnet, daß das Chlorierungsmittel Chlor ist.

3. Verfahren nach Ansprüchen 1 oder 2, dadurch gekennzeichnet, daß der freie Radikale erzeugende Starter elektromagnetische Strahlen sind.

4. Verfahren nach jedem der vorstehenden Ansprüche, dadurch gekennzeichnet, daß das Chlorierungsmittel und 1,1-Difluoroethylen in einem flüssigen Reaktionsmedium in Kontakt miteinander gebracht werden.

5. Verfahren nach jedem der vorstehenden Ansprüche, dadurch gekennzeichnet, daß die Temperatur des flüssigen Reaktionsmediums zwischen −20°C und 100°C beträgt.

6. Verfahren nach Anspruch 4, dadurch gekennzeichnet, daß das flüssige Reaktionsmedium $CF_2ClCH_2Cl$ und $CF_2ClCHCl_2$ in einem Molverhältnis von mindestens 1,5 des Erstegenannten: 1 des Letztgenannten enthält und bei dem das Molverhältnis des zugeführten Chlorierungsmittels, ausgedrückt als zu Verfügung stehendes Chlor zu 1,1-Difluoroethylen von 1,2 bis 2,5 beträgt.

7. Verfahren nach jedem der Ansprüche 4 bis 6, dadurch gekennzeichnet, daß das Molverhältnis des zugeführten Chlorierungsmittels, ausgedrückt als zu Verfügung stehendes Chlor zu 1,1-Difluoroethylen von 1,7 bis 2,1 und die Temperatur des flüssigen Reaktionsmediums von 0°C bis 40°C beträgt, das flüssige Reaktionsmedium $CF_2ClCH_2Cl$ und $CF_2ClCHCl_2$ im Molverhältnis 2:10 enthält und der freie Radikale erzeugende Starter ultraviolettes Licht ist.

8. Verfahren zur Herstellung von 1,1,2-Trichlor-2,2-Difluorethan, gekennzeichnet durch Chlorieren von 1,1-Difluoroethylen mit Chlor in Gegenwart von ultraviolettem Licht bei einer Temperatur von etwa −20°C bis etwa 100°C in einem flüssigen Reaktionsmedium, das $CF_2ClCH_2Cl$ und $CF_2ClCHCl_2$ im Molverhältnis von mindestens etwa 1,5:1 enthält, so daß selektiv 1,1,2-Trichlor-2,2-difluorethane gebildet wird.

9. Verfahren nach jedem der Ansprüche 1 bis 8, dadurch gekennzeichnet, daß während des Ablaufes der Reaktion kontinuierlich oder intermittierend ein Teil des flüssigen Reaktionsmediums entfernt wird und eine an 1,1,2-Trichlor-2,2-difluoroethan reiche Fraktion und eine an $CF_2ClCH_2Cl$ reiche Fraktion daraus erhalten werden und wobei die an $CF_2ClCH_2Cl$ reiche Fraktion in das flüssige Reaktionsmedium zurückgeführt wird.

10. Verfahren nach jedem der vorstehenden Ansprüche, dadurch gekennzeichnet, daß 1,1,2-Trichlor-2,2-difluoroethan mit einer Selktivität von mehr als 63 Mol-% erhalten werden.

11. Verfahren nach Anspruch 10, dadurch gekennzeichnet, daß die Selektivität mindestens 80 Mol-% beträgt.

## Revendications

1. Procédé pour la préparation de trichloro-1,1,2 difluoro-2,2 éthane, qui consiste à mettre du difluoro-1,1 éthylène en contact avec un agent de chloruration en présence d'un initiateur de radicaux libres sous des conditions telles que l'on obtient du trichloro-1,1,2-difluoro-2,2 éthane.

2. Procédé selon la revendication 1, dans lequel l'agent de chloruration est du chlore.

3. Procédé selon la revendication 1 ou la revendication 2, dans lequel l'initiateur de radicaux libres est un rayonnement électromagnétique.

4. Procédé selon l'une quelconque des revendications précédentes, dans lequel on met en contact l'agent de chloruration et le difluoro-1,1 éthylène dans un milieu liquide de réaction.

5. Procédé selon l'une quelconque des revendications précédentes, dans lequel la température du milieu liquide de réaction est comprise entre −20°C et 100°C.

6. Procédé selon la revendication 4, dans lequel le milieu liquide de réaction comprend du $CF_2ClCH_2Cl$ et du $CF_2ClCHCl_2$ dans un rapport molaire qui est d'au moins 1,5 du premier pour 1 du second, et dans lequel le rapport molaire d'introduction de l'agent de chloruration, exprimé en chlore disponible par rapport au difluoro-1,1 éthylène, est de 1,2 à 2,5.

7. Procédé selon l'une quelconque des revendications 4 à 6, dans lequel le rapport molaire d'introduction de l'agent de chloruration, exprimé en chlore disponible par rapport au difluoro-1,1 éthylène, est de 1,7 à 2,1, la température du milieu liquide de réaction est de 0°C à 40°C, ce milieu liquide de réaction comprend du $CF_2ClCH_2Cl$ et du $CF_2ClCHCl_2$ dans un rapport molaire de 2 à 10 et l'initiateur de radicaux libres est de la lumière ultraviolette.

8. Procédé pour la préparation de trichloro-1,1,2-difluoro-2,2 éthane consistant à mettre du difluoro-1,1 éthylène en contact avec du chlore en présence de lumière ultraviolet à une température d'environ −20°C à environ 100°C dans un milieu liquide de réaction comprenant du $CF_2ClCH_2Cl$ et du $CF_2ClCHCl_2$ dans lequel le rapport molaire du $CF_2ClCH_2Cl$ au $CF_2ClCHCl_2$ est d'au mons environ 1,5:1, ce qui permet de produire de manière sélective du trichlor-1,1,2 difluro-2,2 éthane.

9. Procédé selon l'une quelconque des revendications 1 à 8, dans lequel on extrait une partie de milieu liquide de réaction d'une manière continue ou intermittente au cours du déroulement de la réaction et on obtient à partir de celle-ci une fraction riche en trichloro-1,1,2 difluoro-2,2 éthane et une fraction riche en $CF_2ClCH_2Cl$, et dans lequel on recycle la fraction richt en $CF_2ClCH_2Cl$ vers le milieu liquide de réaction.

10. Procédé selon l'une quelconque des revendications précédentes, dans lequel on obtient le trichloro-1,1,2 difluoro-2,2 éthane avec une sélectivité supérieure à 63 moles pour cent.

11. Procédé selon la revendication 10, dans lequel la sélectivité est d'au moins 80 moles pour cent.